# EUROPÄISCHE PATENTSCHRIFT

(11) **EP 2 689 790 B1**
(45) Veröffentlichungstag und Bekanntmachung des Hinweises auf die Patenterteilung: **04.03.2015**
(21) Anmeldenummer: 12005434.1
(22) Anmeldetag: 26.07.2012
(51) Int. Cl.: A61M 1/16

(54) **RO-Anlage und Verfahren zum Desinfizieren von Leitungen der RO-Anlage**
RO device and method for disinfecting lines in an RO device
Installation RO et procédé destiné à désinfecter les conduites de l'installation RO

(43) Veröffentlichungstag der Anmeldung: 29.01.2014
(73) Patentinhaber: Völker, Manfred, 63825 Blankenbach (DE)
(72) Erfinder: Völker, Manfred, 63825 Blankenbach (DE)
(74) Vertreter: Flosdorff, Jürgen

(56) Entgegenhaltungen:
- DE-A1- 10 013 964
- DE-A1- 10 262 036
- DE-A1- 10 319 221
- DE-A1-102009 057 562
- DE-U1-202010 006 823

## Beschreibung

Die Erfindung betrifft eine RO-Anlage, mit der hochreines Wasser vorzugsweise für den Betrieb von Dialysegeräten erzeugt wird. Die RO-Anlage hat ein RO-Filtermodul, das durch eine Membran in einen Primärraum und einen Sekundärraum unterteilt ist, einen atmosphärisch belüfteten Vorlaufbehälter, in den eine Rohwasser-Zuflussleitung einmündet, eine von dem unteren Ende des Vorlaufbehälters zu dem Primärraum führende Leitung, in die eine Pumpe eingeschaltet ist, eine von dem Primärraum weg führende Konzentratleitung, die vorzugsweise zu dem Vorlaufbehälter zurück führt, und eine von dem Sekundärraum des Filtermoduls ausgehende Permeatversorgungsleitung, an die mittels Stichleitung oder sekundärer Ringleitung wenigstens ein Dialysegerät anschließbar ist, wobei ferner in Strömungsrichtung hinter dem Anschluss des wenigstens einen Dialysegerätes eine anschließende Permeatrücklaufleitung zu dem Vorlaufbehälter zurück führt.

Das Leitungssystem einer RO-Anlage muss von Zeit zu Zeit dekontaminiert werden, da sich anderenfalls organische Ablagerungen im Inneren der Leitungen und zugehöriger Funktionseinheiten wie Ventile etc. ablagern und sich Krankheitskeime ansiedeln können, die die Gesundheit von mit den Dialysegeräten behandelten Patienten gefährden könnten. Bisher wird das Leitungssystem von RO-Anlagen in der Praxis mit chemischen Mitteln oder durch Heißreinigung desinfiziert. Die Heißreinigung ist mit einem hohen Energieaufwand verbunden, und bei dem Einsatz von chemischen Reinigungsmitteln muss mit äußerster Sorgfalt dafür gesorgt werden, dass nach der Dekontamination keinerlei chemische Rückstände in dem Leitungssystem und seine Funktionseinheiten zurück bleiben.

Die DE 102 62 036 A1 offenbart eine RO-Anlage mit den Merkmalen des Oberbegriffs des Patentanspruchs 1. Wenn diese Reinstwasserversorgungsanlage im Desinfektionsmodus betrieben wird, muss die Permeaterzeugung unterbrochen werden.

Die in der DE 103 19 221 A1 beschriebene Dialyse-Anlage enthält eine Verbindungsleitung, in der ein Rückschlagventil angeordnet ist, das in einer Richtung einen freien Durchfluss gewährt.

Die DE 10 2009 057 562 A1 offenbart eine RO-Anlage, bei der die Zulaufleitung eine Einrichtung zur physikalischen Desinfektion und zwei parallele Zulaufleitungsabschnitte aufweist, von denen einer über ein Bypassventil mit der Rücklaufleitung verbunden ist.

Der vorliegenden Erfindung liegt die Aufgabe zugrunde, eine RO-Anlage der betrachteten Art anzugeben, bei der ein Desinfektionsmodus bei weiter laufender RO-Anlage durchführbar ist.

Diese Aufgabe wird durch eine RO-Anlage mit den Merkmalen des Patentanspruchs 1 gelöst.

Vorteilhafte Weiterbildungen der Erfindung sind in den anschließenden Unteransprüchen gekennzeichnet.

Außerdem sieht die Erfindung ein Reinigungsverfahren mit den Merkmalen des Anspruchs 7 vor. Vorteilhafte Ausgestaltungen des Verfahrens sind in den anschließenden Unteransprüchen gekennzeichnet.

Die Erfindung sieht vor, dass in die Permeatrücklaufleitung der RO-Anlage eine Zirkulationspumpe und ein elektrochemischer Ozongenerator eingeschaltet sind, und dass die Permeatrücklaufleitung in Strömungsrichtung hinter der Zirkulationspumpe und dem Ozongenerator durch eine Rezirkulationsleitung mit der Permeatversorgungsleitung verbunden ist, wodurch ein geschlossener Kreislauf gebildet werden kann, in dem ozonisiertes Permeat zirkulieren kann, bis alle organischen Fremdsubstanzen in diesem Teil des Leitungssystems durch das Ozon abgetötet oder abgebaut sind. In die Rezirkulationsleitung und in die Permeatrücklaufleitung ist stromabwärts der Abzweigung der Rezirkulationsleitung je ein Ventil eingeschaltet. Das Ventil der Rezirkulationsleitung ist beim normalen Betrieb der RO-Anlage zur Versorgung von angeschlossenen Dialysegeräten geschlossen, während das Ventil in der Permeatrücklaufleitung offen ist, so dass überschüssiges Permeat, das nicht von den Dialysegeräten entnommen wurde, in den Vorlaufbehälter zurück fließen kann. Das Permeat kann alternativ auch in einen Abfluss abgeführt werden.

Wenn hingegen das Ventil in der Permeatrücklaufleitung geschlossen und das Ventil in der Rezirkulationsleitung geöffnet wird, befindet sich die Anlage im Reinigungszustand, in dem der Ozongenerator das durchfließende Permeat mit Ozon anreichert.

Außerdem sieht die Erfindung vor, dass von der Permeatversorgungsleitung stromaufwärts der Einmündung der Rezirkulationsleitung in die Permeatversorgungsleitung ein Permeatrücklaufleitungsstrang abzweigt, der in die Permeatrücklaufleitung stromabwärts des darin angeordneten Ventils einmündet, und dass in dem Permeatrücklaufleitungsstrang ein Druckhalteventil angeordnet ist. Hierdurch ist erreicht, dass die RO-Anlage auch beim Desinfektionsmodus weiterlaufen kann und ohne Unterbrechung Permeat erzeugt, das durch den Permeatrücklaufleitungsstrang in den Vorlaufbehälter der RO-Anlage abfließen kann. Wenn in dem Rezirkulationskreislauf des ozonisierten Permeats kein zusätzliches Permeat benötigt wird, fließt die gesamte Menge des während der Rezirkulation erzeugten frischen Permeats durch das Druckhalteventil zum Vorlaufbehälter ab, in dem sich in dem Rücklaufleitungsstrang vor dem Ventil ein solcher Druck aufbaut, der die Schließkraft des Ventils übersteigt. Anstelle eines derartigen Druckhalteventils kann auch ein durch eine Steuereinrichtung der RO-Anlage z.B. elektrisch gesteuertes Ventil vorgesehen sein.

Das in dem Permeatrezirkulationskreis umlaufende ozonisierte Permeat kann aber auch dazu verwendet werden, das zu dem Wassereingangsventil eines Dialysegerätes führende Leitungssystem zu reinigen, wobei ozonisiertes Permeat bei geöffnetem Spülventil durch die zugehörige Abflussleitung in einen Abfluss abgeführt wird. Dies kann nacheinander bei allen angeschlossenen Dialysegeräten erfolgen.

In diesem Fall fließt frisches, d.h. noch nicht ozonisiertes Permeat in den Rezirkulationskreis ein, da dieser anderenfalls mehr oder weniger leer laufen könnte, wodurch die Funktionsfähigkeit der in dem Rezirkulationskreis angeordneten Pumpe beeinträchtigt werden könnte. Hierbei fließt entweder vorübergehend die gesamte Menge des zugeführten frischen Permeats in den Rezirkulationskreis ein, oder aber die benötigte Teilmenge, während die andere Teilmenge durch den Permeatrücklaufleitungsstrang zum Vorlaufbehälter abfließt.

In weiteren Einzelheiten ist vorgesehen, dass - stromabwärts nach der Einmündung der Rezirkulationsleitung in die Permeatversorgungsleitung ein weiteres Ventil eingeschaltet ist, und dass stromaufwärts des weiteren Ventils eine Verbindungsleitung zu der Permeatrückführleitung abzweigt, in die ein weiteres Druckhalteventil eingeschaltet ist. An der Einmündung der Verbindungsleitung in die Permeatrücklaufleitung oder zwischen dieser Einmündung und dem in die Permeatrücklaufleitung eingeschalteten Ventil zweigt vorteilhafterweise eine zu einem Abfluss führende Abflussleitung ab, in die ein Abflussventil eingeschaltet ist.

Das erfindungsgemäße Verfahren zum Desinfizieren des PermeatLeitungssystems sieht demnach vor, dass das Permeat während eines Reinigungsvorgangs mit Ozon unter Einhaltung einer vorgegeben Ozonkonzentration versehen wird und dass das ozonisierte Permeat in der Permeatleitung zirkuliert, während dessen wenigstens ein Teilstrom des von dem Sekundärraum des Filtermoduls kommenden Permeats zu dem Vorlaufbehälter fließen kann, ohne sich mit dem mit Ozon angereicherten Permeat zu vermischen. Nach einer vorbestimmten Reinigungszeit wird das ozonisierte Permeat in einen Abfluss gespült oder dem Vorlaufbehälter der RO-Anlage zugeführt. In dem letzteren Fall kann das dem Vorlaufbehälter zugeführte ozonisierte Permeat auch den Vorlaufbehälter selbst desinfizieren sowie gegebenenfalls auch die zu dem Filtermodul führende Leitung. Da die Membran des Filters durch Ozon von einer bestimmten Konzentration an beschädigt werden kann, kann auch mit einer geringen Menge Ozon in der Flüssigkeit der Primärraum des Filtermoduls und die vorzugsweise zu dem Vorlaufbehälter zurück führende Konzentratrücklaufleitung gereinigt werden. Hierzu wird an geeigneten Stellen des Leitungssystems die Ozonkonzentration überwacht. Hierzu ist wenigstens eine Messkammer in dem Leitungssystem angeordnet, mit der die in der Flüssigkeit befindliche Menge des Ozons mittels Redoxpotentials oder Ozonsensorik kontrollierbar ist.

Die Menge des von dem Ozongenerator erzeugten Ozons ist nach einem weiteren Vorschlag der Erfindung durch eine ausgewählte angelegte Spannung und/oder ausgewählte Stromstärke steuer- und überwachbar.

Der erfindungsgemäße Rezirkulationskreislauf des Permeatleitungssystems mit den erwähnten Ventilen und der Rezirkulationspumpe kann auch dazu verwendet werden, diesen Bereich der RO-Anlage mit Hilfe eines chemischen Desinfektionsmittels oder auch einer thermischen Einrichtung anstelle des Ozongenerators zu reinigen oder zu desinfizieren. Auch in diesem Fall kann die RO-Anlage beim Reinigungsvorgang weiter laufen und Permeat liefern, wodurch ein Leerlauf der Zirkulationspumpe verhindert ist und die zuführenden Leitungen der angeschlossenen Dialysegeräte zu reinigen bzw. zu desinfizieren sind.

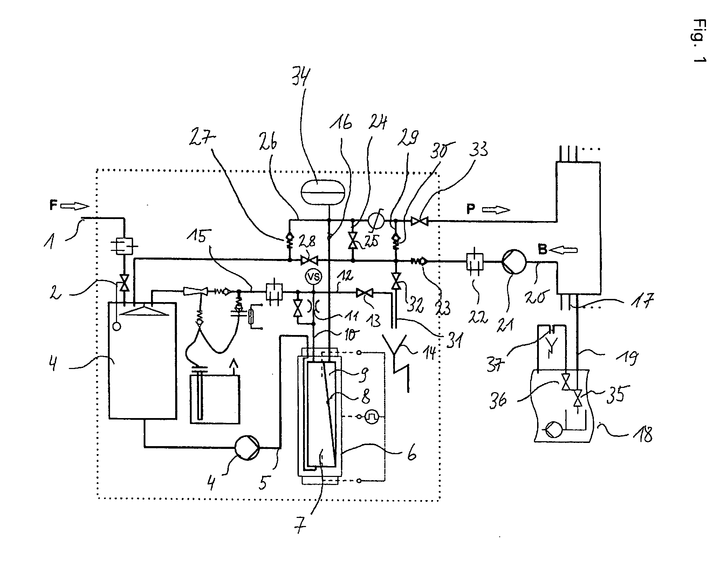

Weitere Einzelheiten der Erfindung ergeben sich aus der nachfolgenden Beschreibung sowie anhand der Zeichnung. Dabei zeigt die einzige Figur eine schematische Darstellung des Leitungssystems einer Ausführungsform der erfindungsgemäßen RO-Anlage mit den zugehörigen Funktionseinheiten.

Es werden nur die Bauteile der Figur beschrieben, die für die vorliegende Erfindung von Bedeutung sind.

Durch eine Leitung 1 wird der RO-Anlage Flüssigkeit zugeführt, das über ein Einlassventil 2 in den Vorlaufbehälter 3 gelangt. Die Flüssigkeit in dem Vorlaufbehälter 3 wird mittels einer Pumpe 4 durch eine Leitung 5 einem RO-Filtermodul 6 zugeführt, dessen Primärraum 7 durch eine Membran 8 von dem Sekundärraum 9 getrennt ist.

Aus dem Primärraum 7 wird Konzentrat durch eine Leitung 10 abgeführt, in der eine Drossel 11 für den im Primärraum 7 herrschenden Druck sorgt. Das Konzentrat kann entweder durch eine Leitung 12, in die ein Ventil 13 eingeschaltet ist, in einen Abfluss 14 abgeführt werden, oder durch eine Konzentratrücklaufleitung 15 in den Vorlaufbehälter 3 zurückgeführt werden.

Aus dem Sekundärraum 9 fließt Permeat durch eine Permeatversorgungsleitung 16 zu Anschlüssen 17 von Dialysegeräten, die in einem dargestellten Beispiel durch jeweils eine Stichleitung 19 mit Permeat versorgt werden können. Eine Versorgung der Dialysegeräte mit hier nicht dargestellten sekundären Leitungen ist ebenfalls möglich. Stromabwärts des letzten Anschlusses eines Dialysegerätes schließt sich an die Permeatversorgungsleitung 16 eine Permeatrücklaufleitung 20 an, die zu dem Vorlaufbehälter 3 zurück führt.

In die Permeatrücklaufleitung 20 sind in Strömungsrichtung hintereinander eine Zirkulationspumpe 21 und ein Ozongenerator 22 eingeschaltet, wobei auf den letzteren ein Druckhalteventil 23 folgt. Eine andere Lage der Zirkulationspumpe und der Ozonzelle z.B. in die Permeatvorlaufleitung oder die Verwendung einer thermischen- bzw. chemischen Desinfektions-, Reinigungseinrichtung liegt ebenfalls im Rahmen der Erfindung.

Eine Rezirkulationsleitung 24 mit darin angeordnetem Ventil 25 verbindet die Permeatrücklaufleitung 20 mit der Permeatversorgungsleitung 16, so dass ein Rezirkulationskreis gebildet ist, in dem ozonisiertes Permeat zirkulieren kann.

Von der Permeatversorgungsleitung 16 zweigt in Strömungsrichtung vor der Rezirkulationsleitung 24 ein Permeatrücklaufleitungsstrang 26 ab, in den ein Druckhalteventil 27 eingeschaltet ist, welches während der Desinfektion bzw. Reinigung den Ringvordruck bestimmt und über das überschüssiges Permeat abfließt. Der Permeatrücklaufleitungsstrang 26 mündet in die Permeatrücklaufleitung 20 stromabwärts eines darin eingeschalteten Ventils 28 ein.

Stromabwärts der Einmündung der Rezirkulationsleitung 24 in die Permeatversorgungsleitung 16 zweigt eine Verbindungsleitung 29 mit einem Druckhalteventil 30 ab, die in die Permeatrücklaufleitung 20 einmündet und von dort als Abflussleitung 31 mit einem Absperrventil 32 zu dem Abfluss 14 führt. Stromabwärts der Verbindungsleitung 29 befindet sich in der Permeatversorgungsleitung 16 ein weiteres Ventil 33, welches durch die Leitfähigkeits-Temperaturmessung 34 gesteuert werden kann.

Die Stichleitung 19 führt zu einem Wassereingangsventil 35 eines Dialysegerätes 18. Unmittelbar vor dem Wassereingangsventil 35 zweigt eine zu einem Abfluss führende Abflussleitung 37 mit einem Spülventil 36 ab.

| Legende | Bezeichnung |
|---|---|
| 1 | Flüssigkeitszuführung |
| 2 | Einlassventil |
| 3 | Vorlaufbehälter |
| 4 | Pumpe |
| 5 | Zulaufleitung Filtermodul |
| 6 | Filtermodul |
| 7 | Primärraum |
| 8 | Membran |
| 9 | Sekundärraum |
| 10 | Konzentratleitung |
| 11 | Drossel |
| 12 | Leitung |
| 13 | Konzentratabflussventil |
| 14 | Abfluss |
| 15 | Konzentratrücklaufleitung |
| 16 | Permeatzulaufleitung |
| 17 | Anschlüsse Dialysegerät |
| 18 | Dialysegerät |
| 19 | Stichleitung |
| 20 | Permeatrücklaufleitung |
| 21 | Zirkulationspumpe |
| 22 | Elektrochemischer Ozongenerator |
| 23 | Druckhalteventil |
| 24 | Rezirkulationsleitung |
| 25 | Rezirkulationsventil |
| 26 | Teil Permeatrücklauf |
| 27 | Druckhalteventil |
| 28 | Rücklaufsperrventil |
| 29 | Verbindungsleitung |
| 30 | Druckhalteventil |
| 31 | Abflussleitung |
| 32 | Ringabflussventil |
| 33 | Ringdruck-Schutzventil |
| 34 | Temperatur- Leitfähigkeitsüberwachung |
| 35 | Wassereingangsventil Dialysegerät |
| 36 | Dialysegerät-Spülventil |
| 37 | Abflussleitung |

## Patentansprüche

1. RO-Anlage mit einem RO-Filtermodul (6), das durch eine Membran (8) in einen Primärraum (7) und einen Sekundärraum (9) unterteilt ist, mit einem atmosphärisch belüfteten Vorlaufbehälter (3), in den eine Rohwasserzuflussleitung (1) einmündet, wobei von dem unteren Ende des Vorlaufbe-hälters (3) eine Leitung (5), in die eine Pumpe (4) eingeschaltet ist, zu dem Primärraum (7) führt,
wobei von dem Primärraum (7) eine Konzentratleitung (10) weg führt,
wobei von dem Sekundärraum (9) eine Permeatversorgungsleitung (16) ausgeht, an die mittels Stichleitung (17) oder sekundärer Ringleitung wenigstens ein Dialysegerät (18) anschließbar ist,
wobei ferner in Strömungsrichtung hinter dem wenigstens einen Dialysegerät (18) eine anschließende Permeatrücklaufleitung (20) zu dem Vorlaufbehälter (3) zurückführt,
wobei in die Permeatrücklaufleitung (20) eine Zirkulationspumpe (21) und ein elektrochemischer Ozongenerator (22) eingeschaltet sind,
und wobei die Permeatrücklaufleitung (20) in Strömungsrichtung hinter der Zirkulationspumpe (21) und dem Ozongenerator (22) durch eine Rezirkulationsleitung (24) mit der Permeatversorgungsleitung (16) verbunden ist, wobei in die Rezirkulationsleitung (24) und in die Permeatrücklaufleitung (20) stromabwärts der Abzweigung der Rezirkulationsleitung (24) je ein Ventil (25,28) eingeschaltet ist,
**dadurch gekennzeichnet,**
**dass** von der Permeatversorgungsleitung (16) stromaufwärts der Einmündung der Rezirkulationsleitung (24) in die Permeatversorgungsleitung (16) ein Permeatrücklaufleitungsstrang (26) abzweigt, der in die Permeatrücklaufleitung (20) stromabwärts des darin angeordneten Ventils einmündet, und
**dass** in dem Permeatrücklaufleitungsstrang (26) ein Druckhalteventil (27) angeordnet ist,
so dass die RO-Anlage auch beim Desinfektionsmodus weiter laufen kann und ohne Unterbrechung Permeat erzeugt und entweder die gesamte Permeatmenge oder ein Permeatteilstrom zurück zum Vorlaufbehälter (3) fließt.

2. RO-Anlage nach Anspruch 1, **dadurch gekennzeichnet,dass** stromabwärts nach der Einmündung der Rezirkulationsleitung (24) in die Permeatversorgungsleitung (16) ein weiteres Ventil (33) eingeschaltet ist.

3. RO- Anlage nach Anspruch 2, **dadurch gekennzeichnet,dass** zwischen der Einmündung der Rezirkulationsleitung (24) in die Permeatversorgungsleitung (16) und dem weiteren Ventil (33) eine Verbindungsleitung (29) zu der Permeatrückführleitung (20) abzweigt, in die ein weiteres Druckhalteventil (30) eingeschaltet ist.

4. RO-Anlage nach Anspruch 3, **dadurch gekennzeichnet, dass** an der Einmündung der Verbindungsleitung (29) in die Permeatrücklaufleitung (20) oder zwischen dieser und deren Ventil (28) eine zu einem Abfluss (14) führende Abflussleitung (31) abzweigt, in die ein Abflussventil (32) eingeschaltet ist.

5. RO-Anlage nach Anspruch 1 bis 4, **dadurch gekennzeichnet, dass** die Menge des von dem Ozongenerator (22) erzeugten Ozons durch eine ausgewählte angelegte Spannung und/oder ausgewählte Stromstärke steuerbar ist.

6. RO-Anlage nach den Ansprüchen 1 bis 5, **dadurch gekennzeichnet, dass** sich in der Permeatversorgungsleitung (16) oder der Permeatrücklaufleitung eine Messkammer (34) zur Überwachung der Ozon-Konzentration befindet, mit der die in der Flüssigkeit befindliche Menge des Ozons mittels Redoxpotentials oder Ozonsensorik kontrollierbar ist.

7. Verfahren zum Desinfizieren von Leitungen einer RO-Anlage gemäß einem der Ansprüche 1-6, wobei das Permeat während eines Reinigungsvorgangs der RO-Anlage mit Ozon unter Einhaltung einer vorgegebenen Ozonkonzentration versehen wird und das ozonisierte Permeat in der Permeatleitung (16,20) zirkuliert, während dessen die RO-Anlage weiter läuft und ohne Unterbrechung Permeat erzeugt und entweder die gesamte Permeatmenge oder wenigstens ein Teilstrom des von dem Sekundärraum (9) des Filtermoduls (6) kommenden Permeats zu dem Vorlaufbehälter (3) fließt, ohne sich mit dem mit Ozon angereicherten Permeat zu vermischen, undwobei nach einer vorbestimmten Zeit das ozonisierte Permeat in einen Abfluss (14) gespült oder dem Vorlaufbehälter (3) der RO-Anlage zugeführt wird.

8. Verfahren nach Anspruch 7, **dadurch gekennzeichnet, dass** das dem Vorlaufbehälter (3) zugeführte ozonisierte Permeat den Vorlaufbehälter (3), und vorzugsweise die zum Filtermodul führende Leitung (5), den Primärraum (7) des Filtermoduls (6) und die Konzentratrücklaufleitung (10) desinfiziert.

9. Verfahren nach Anspruch 7 oder 8, **dadurch gekennzeichnet, dass** das Permeat-Ozon-Gemisch bis zu dem Wassereingangsventil (35) eines Dialysegerätes (18) geleitet und bei geöffnetem Spülventil (36) durch die zugehörige Abflussleitung (37) abgeführt wird, während ein Teilstrom des aus dem Sekundärraum (9) austretenden Permeats in die Permeatzulaufleitung (16) fließt.

## Claims

1. An RO system with an RO filter module (6), which is divided by a membrane (8) into a primary space (7) and a secondary space (9), a feed tank (3), which is vented to atmosphere and communicating with which is a raw water supply conduit (1), wherein leading to the primary space (7) from the lower end of the feed tank (3) there is a conduit (5), connected into which there is a pump (4), wherein leading away from the primary space (7) there is a concentrate conduit (10), wherein starting from the secondary space (9) there is a permeate supply conduit (16), to which at least one dialysis device (18) is connectable by means of a tap conduit (17) of a secondary ring conduit, wherein further a subsequent permeate return conduit (20) leads back after the at least one dialysis device (18) in the flow direction to the feed tank (3), wherein connected into the permeate return conduit (20) there are a circulation pump (21) and an electrochemical ozone generator (22), and wherein the permeate return conduit (20) is connected after the circulation pump (21) and the ozone generator (22) in the flow direction to the permeate supply conduit (16) by a recirculation conduit (24), wherein connected into the recirculation conduit (24) and into the permeate return conduit (20) downstream of the junction with the recirculation conduit (24) there are respective valves (25, 28) **characterised in that** branching off from the permeate supply conduit (16) upstream of the communication of the recirculation conduit (24) with the permeate supply conduit (16) there is a permeate return conduit section (26), which communicates with the permeate return conduit (20) downstream of the valve arranged in it and that arranged in the permeate return conduit section (26) there is a pressure retention valve (27) so that the RO system can continue to operate even during the disinfection mode and produces permeate without interruption and either the entire permeate volume or a proportion of it flows back to the feed tank.

2. An RO system as claimed in claim 1, **characterised in that** connected downstream of the communication of the recirculation conduit (24) with the permeate supply conduit (16) there is a further valve (33).

3. An RO system as claimed in claim 2, **characterised in that** branching off between the communication of the recirculation conduit (24) with the permeate supply conduit (16) and the further valve (33) there is a connecting conduit (29) to the permeate return conduit (20), connected into which there is a further pressure retention valve (30).

4. An RO system as claimed in claim 3, **characterised in that** branching off at the communication of the connecting conduit (20) with the permeate return conduit (20) or between the latter and its valve (28) there is a drain conduit (31), which leads to an outlet (14) and connected into which there is a drain valve (32).

5. An RO system as claimed in claims 1 to 4, **characterised in that** the amount of the ozone generated by the ozone generator (22) is controllable by means of a selected applied voltage and/or selected current.

6. An RO system as claimed in claims 1 to 5, **characterised in that** situated in the permeate supply conduit (16) or the permeate return conduit there is a measuring chamber (34) for monitoring the ozone concentration, with which the amount of the ozone in the liquid may be checked by means of the redox potential or an ozone sensor system.

7. A method of disinfecting conduits of an RO system as claimed in one of claims 1-6, wherein the permeate is provided during a cleaning process of the RO system with ozone whilst maintaining a predetermined ozone concentration and the ozonised permeate circulates in the permeate conduit (16, 20), during which the RO system continues to operate and produces permeate without interruption and either the entire volume of permeate or at least a proportion of the permeate coming from the secondary space (9) of the filter module (6) flows to the feed tank (3) without mixing with the permeate enriched with ozone, and whereby after a predetermined time the ozonised permeate is flushed into an outlet (14) or is fed to the feed tank (3) of the RO system.

8. A method as claimed in claim 7, **characterised in that** the ozonised permeate supplied to the feed tank (3) disinfects the feed tank (3) and preferably the conduit (5) leading to the filter module, the primary space (7) of the filter module (6) and the concentrate return conduit (10).

9. A method as claimed in claim 7 or 8, **characterised in that** the permeate-ozone mixture is conducted to the water inlet valve (35) of a dialysis device (18) and is discharged, with the flushing valve (36) opened, through the associated drain conduit (37) whilst a proportion of the permeate flowing out of the secondary space (9) flows into the permeate supply conduit (16).

## Revendications

1. Installation RO comprenant un module de filtrage RO (6), qui est divisé par une membrane (8) en un espace primaire (7) et un espace secondaire (9), comprenant un réservoir de décantation (3) à aération atmosphérique, dans lequel débouche une conduite d'arrivée d'eau brute (1), sachant qu'une conduite (5) dans laquelle est montée une pompe (4) conduit à l'espace primaire (7) depuis l'extrémité inférieure du réservoir de décantation (3),
sachant qu'une conduite de concentrat (10) s'éloigne de l'espace primaire (7),
sachant qu'une conduite d'alimentation en perméat (16) part depuis l'espace secondaire (9), à laquelle au moins un appareil de dialyse (18) peut être raccordé au moyen d'une conduite de dérivation (17) ou d'une conduite annulaire secondaire,
sachant en outre qu'une conduite de retour de perméat (20) se raccordant revient au réservoir de décantation (3) dans le sens de l'écoulement, derrière l'appareil de dialyse (18) au moins au nombre de un,
sachant qu'une pompe de circulation (21) et un générateur d'ozone (22) électrochimique sont montés dans la conduite de retour de perméat (20),
et sachant que la conduite de retour de perméat (20) est reliée, dans le sens de l'écoulement derrière la pompe de circulation (21) et le générateur d'ozone (22), par une conduite de recirculation (24), à la conduite d'alimentation en perméat (16),
sachant que respectivement une soupape (25, 28) est montée dans la conduite de recirculation (24) et dans la conduite de retour de perméat (20) en aval de l'embranchement de la conduite de recirculation (24),
**caractérisée en ce que**
un brin de conduite de retour de perméat (26) forme un embranchement de la conduite d'alimentation en perméat (16), en amont de l'embouchure de la conduite de recirculation (24) dans la conduite d'alimentation en perméat (16), lequel débouche dans la conduite de retour de perméat (20) en aval de la soupape disposée dans cette dernière, et
une soupape de maintien de pression (27) est disposée dans le brin de conduite de retour de perméat (26),
de sorte que l'installation RO peut continuer de fonctionner également en mode de désinfection et produit sans interruption du perméat ou de sorte que la quantité totale de perméat ou un flux partiel de perméat revient par écoulement en direction du réservoir de décantation (3).

2. Installation RO selon la revendication 1, **caractérisée en ce qu'**une autre soupape (33) est montée dans la conduite d'alimentation en perméat (16) en aval après l'embouchure de la conduite de recirculation (24).

3. Installation RO selon la revendication 2, **caractérisée en ce qu'**une conduite de raccordement (29) menant à la conduite de retour de perméat (20) forme un embranchement entre l'embouchure de la conduite de recirculation (24) dans la conduite d'alimentation en perméat (16) et l'autre soupape (33), une autre soupape de maintien de pression (30) étant montée dans la conduite de retour de perméat.

4. Installation RO selon la revendication 3, **caractérisée en ce qu'**une conduite d'évacuation (31) menant à une évacuation (14) forme un embranchement au niveau de l'embouchure de la conduite de raccordement (29) dans la conduite de retour de perméat (20) ou entre cette dernière et la soupape (28) de cette dernière, une soupape d'évacuation (32) étant montée dans ladite conduite d'évacuation (31).

5. Installation RO selon la revendication 1 à 4, **caractérisée en ce que** la quantité d'ozone produite par le générateur d'ozone (22) peut être commandée par une tension choisie appliquée et/ou par une intensité de courant choisie.

6. Installation RO selon les revendications 1 à 5, **caractérisée en ce qu'**une chambre de mesure (34) servant à surveiller la concentration d'ozone se trouve dans la conduite d'alimentation en perméat (16) ou dans la conduite de retour de perméat, avec laquelle chambre de mesure la quantité d'ozone se trouvant dans le liquide peut être contrôlée au moyen du potentiel d'oxydo-réduction ou d'un système de détection d'ozone.

7. Procédé servant à désinfecter des conduites d'une installation RO selon l'une quelconque des revendications 1 à 6, sachant que le perméat est fourni, au cours d'une opération de nettoyage de l'installation RO, avec de l'ozone pour maintenir une concentration d'ozone spécifiée, et en ce que le perméat ozonisé circule dans la conduite de perméat (16, 20) au cours de laquelle opération de nettoyage, l'installation RO continue de fonctionner et produit sans interruption du perméat ou la quantité totale de perméat ou au moins un flux partiel du perméat provenant de l'espace secondaire (9) du module de filtrage (6) s'écoule en direction du réservoir de décantation (3) sans se mélanger au perméat enrichi en ozone, et sachant que le perméat ozonisé est nettoyé par rinçage dans une évacuation (14) ou est amené au réservoir de décantation (3) de l'installation RO après une durée prédéterminée.

8. Procédé selon la revendication 7, **caractérisé en ce que** le perméat ozonisé amené au réservoir de décantation (3) désinfecte le réservoir de décantation (3) et, de préférence, la conduite (5) menant au module de filtrage, l'espace primaire (7) du module de filtrage (6) et la conduite de retour de concentrat (10).

9. Procédé selon la revendication 7 ou 8, **caractérisé en ce que** le mélange d'ozone et de perméat est acheminé jusqu'à la soupape d'entrée d'eau (35) d'un appareil de dialyse (18) et est évacué par la conduite d'évacuation (37) associée lorsque la soupape de rinçage (36) est ouverte, tandis qu'un flux partiel du perméat sortant de l'espace secondaire (9) s'écoule dans la conduite d'arrivée de perméat (16).
